# EUROPEAN PATENT APPLICATION

(11) **EP 4 245 346 A1**
(43) Date of publication of application: **20.09.2023**
(21) Application number: 22162234.3
(22) Date of filing: 15.03.2022
(51) Int. Cl.: A61M 21/00, A61M 21/02, G06F 3/01, G16H 20/00, G02B 27/01

(54) **THERAPEUTIC SYSTEM FOR THE IMPLEMENTATION OF A THERAPEUTIC METHOD FOR PAIN RELIEF**

(71) Applicant: Lucine, 33300 Bordeaux (FR)
(72) Inventor: COTTY ESLOUS, Maryne, 33500 Libourne (FR)
(74) Representative: Aquinov

(57) **Abstract**

The invention concerns a therapeutic system for the implementation of a therapeutic method for pain relief, comprising a virtual reality head mounted device having at least a head-mounted display and stereo headphones; said system comprising at least a storage medium and a controlling unit of the virtual reality head mounted device, wherein at least a first, a second and a third therapeutic components are stored on the storage medium; wherein:
a. said first therapeutic component comprises an immersive video stream intended to generate a virtual reality environment in which a patient has to be immersed;
b. said second therapeutic component comprises a first soundtrack comprising a first and a second successive sequences of binaural beats, the beats frequencies differences being different from one binaural beats sequence to the other;
c. said third therapeutic component comprises a second soundtrack comprising a spoken hypnosis script;
and wherein, following the reception of a trigger instruction of the therapeutic method, said controlling unit is configured to simultaneously stream the first, the second and the third therapeutic components through the virtual reality head mounted device.

## Description

### FIELD OF INVENTION

The present invention relates to the field of digital therapeutics (DTx) and digital therapeutic systems.

### BACKGROUND OF INVENTION

Chronic pain is a pathological condition defined as a persistent or recurrent pain, lasting for at least three months. It can lead to critical dysfunctions in both peripherical and central nervous systems, including grey and white matter loss, increase or decrease of the activity in major cerebral areas, alterations of synaptic neurotransmission, etc. In addition, chronic pain can severely affect the lifestyle of patients suffering from it: from nutrition and physical activity, to sleep disorders and mental wellbeing. Chronic pain can appear through aging, but also emerges in patients presenting specific diseases, such as in women diagnosed with endometriosis.

Endometriosis is a pathology that has seen its study exponentially increased in the last century. It is characterized by a lesioned endometrial tissue migrating outside the uterus, mainly on the pelvic peritoneum, ovaries, and rectovaginal septum. In rarer cases, it might also reach deeper tissues, such as the diaphragm pleura and the pericardium. Endometriosis is a multifactorial disease, resulting from the combined action of genetic and environmental factors. In this manner, abnormalities of the immune system or the angiogenesis, as well as biochemical factors, play a crucial role in specific biological pathways promoting the growth of endometrial lesions. The lesions of the endometrial tissue and their migration are major contributors to chronic pelvic pain and infertility among women.

The mean prevalence of endometriosis in women with chronic pelvic pain has been estimated at 70 ± 3 %, but might varies from 2 % to 93 % depending on the studies. Similarly, this percentage in women with acute pelvic pain is reaching over 33 %. An annual incidence of about 1/1000 has been reported in women aged 15-49. Among the numerous painful symptoms of endometriosis, the most common ones are dysmenorrhea and profound dyspareunia, appearing in nearly 80% and 30% of the patients, respectively. Dyschesia/dysuria or intermenstrual pelvic pain are less frequently reported, and usually associated with rectal and bladder lesions or ovulation, respectively. All these pains can be nociceptive, neuropathic, or nociplastic (a combination of the previous ones); endometriosis frequently generating all three types of pain.

Current management of endometriosis pain involves pharmacological and/or surgical treatments. The goals of these medical therapies are, for instance, to inhibit ovulation, suppress menstruation, or achieve a stable steroidal hormonal environment. These are based on the concept that the response of the eutopic and ectopic endometrium is substantially similar.

Nevertheless, besides not being adapted for women willing to get pregnant, the pharmacological approaches are not always sufficient to reduce endometriosis pain. Thus, patients who are ineligible, refractory, or not fully responsive to drug treatments, as well as those ineligible for surgical treatments, lack of pain-reliever solutions. Moreover, recurrence of pain after surgery, with or without additional hormonal therapy, is common.

There is thus a need for the development of non-pharmacological and non-surgical alternatives for managing and alleviating pain, in particular endometriosis pain.

Digital therapeutics (DTx) are an evolving field, defined as treatments or therapies that utilize digital products rather than pharmacological products to improve health outcomes. DTx have been or are being developed for treating or managing a variety of conditions, including type II diabetes, Alzheimer's disease, dementia, congestive heart failure, chronic obstructive pulmonary disease, asthma, lung disease, obesity, substance abuse, attention deficit hyperactivity disorder, insomnia, hypertension, anxiety, depression, etc.

Here, the Inventors have developed a digital therapeutic solution that proved efficient at alleviating pain, in particular chronic pelvic pain.

### INVENTION

For this purpose, the subject of the invention is a therapeutic system for the implementation of a therapeutic method for pain relief, comprising a virtual reality head mounted device having at least a head-mounted display and stereo headphones; said system comprising at least a storage medium and a controlling unit of the virtual reality head mounted device, wherein at least a first, a second and a third therapeutic components are stored on the storage medium; wherein:
a. said first therapeutic component comprises an immersive video stream intended to generate a virtual reality environment in which a patient has to be immersed;
b. said second therapeutic component comprises a first soundtrack comprising a first and a second successive sequences of binaural beats, the beats frequencies differences being different from one binaural beats sequence to the other;
c. said third therapeutic component comprises a second soundtrack comprising a spoken hypnosis script;
and wherein, following the reception of a trigger instruction of the therapeutic method, said controlling unit is configured to simultaneously stream the first, the second and the third therapeutic components through the virtual reality head mounted device.

The Inventors have shown, through experiments, that the therapeutic system according to the invention can implement an efficient digital therapeutic treatment for women suffering from pelvic pain related with endometriosis. Compared with known therapeutic methods, as digital control implemented with a projection of an image on a tablet associated with nature sounds, it has been shown that pain perception is superiorly reduced with the therapeutic system according to the invention. The invention is particularly outstanding due to the implementation of two successive, i.e. immediately successive or time-spaced, sequences of different binaural beats. It has been shown that the first sequence has a specific impact on the hypnosis induction phase, while the second sequence strongly helps the patient to focus on the suggestions provided during the hypnosis injunction phase. These joint effects are enhanced by the immersion of the patient in the virtual reality environment. The reduction of pain perception can thus be enhanced, and even doubled, as the invention combines virtual reality immersion, binaural beats and hypnosis and can therefore act on different components of pain. In addition, it has been shown that the decrease in pain caused by the digital system according to the invention is immediate and persists up to 4 hours post-treatment.

In the context of the present specification, unless expressly provided otherwise, a "head mounted display" may refer, but is not limited to, to a virtual reality headset or to any suitable device intended to be worn by a user's head and covering the user's eyes, the device being provided with an electronic display, one or more position and/or motion sensors and a controlling unit. The electronic display might comprise a single electronic display or multiple electronic displays (e.g., a display for each eye of a user). The device might be designed to limit the peripheral vision of the user with using blinders on the sides and top of the device. The controlling unit might be embedded into the device or it might reside on separate hardware, such as a dedicated computer, a smartphone, or a distant server.

In the context of the present specification, unless expressly provided otherwise, the expression "storage medium" is intended to include random access memory (RAM) as dynamic random-access memory (DRAM) or static random-access memory (SRAM), read-only memory (ROM), and/or other types of memory. It might be embedded into the head mounted display or it might reside on separate hardware, such as a dedicated computer, a smartphone, or a distant server.

In the context of the present specification, unless expressly provided otherwise, the expression "immersive video stream" may refer, but is not limited to, to a 360°-degree video, which comprises views of a same scene or a same environment for multiple directions and, optionally, from multiple positions, at a same time. It might be based on live captured images of a real scene or based on computer generated images.

In the context of the present specification, unless expressly provided otherwise, the expression "virtual reality environment" may refer, but is not limited to, to an immersive environment. A virtual reality device generates, from an immersive video stream, images that simulate a subject's physical presence in a virtual reality environment. It is typically possible to explore a virtual reality environment to some extent, e.g., by remaining at a given position in the environment and looking around, or by changing position in the environment.

In the context of the present specification, unless expressly provided otherwise, the expression "binaural beats" may refer, but is not limited to, to an auditory illusion perceived when two different pure tone sine waves, both with frequencies typically lower than 1500 Hz, and with 1 Hz to 300 Hz or more difference between them, are dichotically presented to a subject (i.e., one through each ear). For illustration purposes, a pure tone of 450 Hz can be presented in one ear of the subject and the same pure tone but shifted to 460 Hz can be presented to the other ear of the subject. The subject will not perceive the two tones separately; instead, a single tone will be perceived, whose lateralization moves across the head at a rate of 10 Hz, from centered, to one side, then from the other side back to the center, and so on. Binaural beats are categorized as delta, theta, alpha, beta and gamma, depending on the difference of sine wave frequency presented in each ear of the subject

In the context of the present specification, unless expressly provided otherwise, the expression "spoken hypnosis script" may refer, but is not limited to, to a speech comprising hypnotic language which, when recited to a subject, induces a hypnotic state or a state of trance of said subject. Hypnosis scripts are typically divided into several parts, including (i) hypnosis induction (i.e., pre hypnotic instructions or suggestions given to the subject to enter a hypnotic state), (ii) hypnosis injunctions (i.e., hypnotic instructions or suggestions given to the subject to elicit specific outcomes), and (iii) hypnosis exit (i.e., post hypnotic instructions or suggestions given to the subject to exit the hypnotic state)

According to the invention, a "simultaneously stream of therapeutical components" means that the components are streamed at least partially astride with each other. For instance, each binaural beats sequence might be streamed simultaneously with a part of the spoken hypnosis script and/or with a part of the immersive video stream. For instance, the immersive video stream might start and end simultaneously with the second soundtrack, while the first soundtrack might start after and end before the second soundtrack and the immersive video stream. In case of two video therapeutic components being simultaneously streamed, one component might be superimposed with the other component, for instance with being displayed in the foreground of the other component. In case of two sound therapeutic components being simultaneously streamed, the two components might be mixed, evenly or not.

According to an embodiment, the first sequence of binaural beats comprises or consists of an alpha binaural beats sequence comprising two dichotic pure-tone sine waves, with a frequency difference ranging from about 9 to about 14 Hz; and the second sequence of binaural beats comprises or consists of a theta binaural beats sequence following said alpha binaural beats sequence and comprising two dichotic pure-tone sine waves, with a frequency difference ranging from about 4 to about 8 Hz.

In some embodiment, one or each of the alpha binaural beats sequence and the theta binaural beats sequence comprises or consists of two dichotic soundtracks, each comprising or consisting of a pure tone sine wave with a frequency ranging from about 100 Hz to about 1000 Hz, especially from about 450 to about 500 Hz. More particularly, the left soundtrack of the alpha binaural beats sequence might consist of a pure tone sine wave with a frequency of about 466 Hz while the right soundtrack of the alpha binaural beats sequence might consist of a pure tone sine wave with a frequency of about 456 Hz ; and the left soundtrack of the theta binaural beats sequence might consist of a pure tone sine wave with a frequency of about 466 Hz while the right soundtrack of the theta binaural beats sequence might consist of a pure tone sine wave with a frequency of about 460 Hz.

In some embodiments, the alpha binaural beats sequence and/or the beta binaural beats sequence lasts of a period of time ranging from about 120 to about 360 seconds, especially of 240 seconds.

In some embodiment, the theta binaural beats sequence is time-spaced from the alpha binaural beats sequence of a period of time from about 240 seconds to about 480 seconds, especially of 360 seconds.

According to an embodiment, the second soundtrack comprises:
a. a first hypnosis induction block of predefined sentences spoken by a human voice;
b. a second hypnosis injunction block of predefined sentences spoken by a human voice; and
c. a third hypnosis exit block of predefined sentences spoken by a human voice.

In some embodiments, each sentence of the second hypnosis injunction block, and optionally of the first hypnosis induction block, is time-spaced from the previous sentence of the block of a period of time ranging from about 2 seconds to 5 seconds.

In some embodiments, the first hypnosis induction block lasts of a period of time ranging from about 40 seconds to about 180 seconds, especially of about 120 seconds or 130 seconds.

In some embodiments, the second hypnosis injunction block lasts of a period of time ranging from about 600 seconds to about 1200 seconds, especially of about 950 seconds or 1080 seconds.

In some embodiments, the third hypnosis exit block lasts of a period of time ranging from about 20 seconds to about 240 seconds, especially of about 120 seconds or 180 seconds.

In some embodiments, the second soundtrack is divided into subsequences, including several or all, either unique or multiple, of the following: a spoken welcoming subsequence, a spoken induction subsequence, a spoken dissociation subsequence, a spoken questioning subsequence (e.g., about bodily sensations), a spoken sensory openness subsequence, a spoken post hypnotic suggestion subsequence

In some embodiments, the second hypnosis injunction block comprises one or more questions or remarks about the user feelings and/or bodily sensations.

In some embodiments, the second hypnosis injunction block comprises one or more requests to the user asking him to breath in and out. Preferably, said requests might be streamed after the end of the streaming of the second sequence of binaural beats.

According to an embodiment, each block is spoken by a same woman characterized by a voice frequency ranging from about 150 to about 300 Hz. By "voice frequency", it is meant a fundamental frequency of a speech tone. As an alternative, each block is spoken by a same man characterized by a voice frequency ranging from about 75 to about 150 Hz. As an other alternative, some blocks might be spoken by a man and some other blocks might be spoken by a woman.

According to an embodiment, the voice rhythm and/or the voice pitch and/or the voice loudness and/or the voice harmonic to noise ratio, and optionally the voice frequency and/or the period of time spacing two sentences decreases along the second hypnosis injunction block.

In some embodiments, the voice rhythm and/or the voice pitch and/or the voice loudness and/or the voice harmonic to noise ratio, and optionally the voice frequency and/or the period of time spacing two sentences continuously decreases along the second hypnosis injunction block. As a variant, the voice rhythm and/or the voice pitch and/or the voice loudness and/or the voice harmonic to noise ratio, and optionally the voice frequency and/or the period of time spacing two sentences decreases for at least one, especially each, subsequences of the second hypnosis injunction block.

According to an embodiment, the first sequence of binaural beats is to be streamed astride the streaming of the first hypnosis induction block and the second hypnosis injunction block; and the second sequence of binaural beats is to be streamed during the streaming of the second hypnosis injunction block. In other words, a beginning part of the first sequence of binaural beats is to be streamed concomitantly with the end of the streaming of the first hypnosis induction block and an end part of the first sequence of binaural beats is to be streamed concomitantly with the beginning of the streaming of the second hypnosis injunction block, while the whole second sequence of binaural beats is to be streamed during the streaming of the second hypnosis injunction block.

According to an embodiment, the immersive video stream comprises a sequence of images representing said virtual reality environment and in which at least a subset of images represents the appearance and the disappearance of a graphical trigger element in said virtual reality environment.

In some embodiments, said virtual reality environment might be a natural environment. Natural environments include, but are not limited to, bodies of waters and their shores, such as, e.g., oceans, seas, rivers, lakes, ponds, lagoons, coves, fjords, bays, and the like; meadows, fields, grasslands and the like; forests; hills, mountains; and combinations thereof. As a variant, said virtual reality environment might be an imaginary or fantasy environment. By "imaginary or fantasy environment", it is meant an environment which is not natural, i.e., unreal.

It is desirable in particular that graphical trigger elements appear and disappear in the virtual reality environment to avoid any monotony and boredom of a subject immersed in the virtual reality environment. An example of such trigger element is a shooting star sailing through the sky, a cloud of fireflies flying away, a bubble that bursts, an appearance of a rainbow or a flower that opens.

In some embodiments, said subset of images is to be streamed during the streaming of the first or the second sequence of binaural beats, and, if applicable, of the second hypnosis injunction block. If so, the second hypnosis injunction block might include one or more sentences inviting the user to look at an area of the virtual reality environment, said graphical trigger elements appearing in said area.

According to an embodiment, the first or the second sequence of binaural beats is to be streamed simultaneously with the streaming of said subset of images. If so, the streaming of the first or the second sequence of binaural beats might be longer than the streaming of the subset of images.

According to an embodiment, the immersive video stream comprises a first sequence of images representing said virtual reality environment with a first dominant color and a second sequence of images, immediately following the first sequence of images and representing said virtual reality environment with a second dominant color distinct from the first dominant color, and the first sequence of binaural beats is to streamed with or after the beginning of the streaming the second sequence of image. Preferably, said second sequence of images might comprise said subset of images represents the appearance and the disappearance of a graphical trigger element in said virtual reality environment.

In some embodiments, said first sequence of images might be streamed astride the first hypnosis injunction block and the second hypnosis induction block, the transition from the first sequence of images towards the second sequence of images occurring during the streaming of the second hypnosis induction block.

In some embodiment, the immersive video stream comprises a third sequence of images, immediately following the second sequence of images and representing said virtual reality environment with a third dominant color distinct from the first and the second dominant colors, and the third sequence of images is to be streamed after the end of the streaming the second sequence of binaural beats.

In some embodiments, said third sequence of images might be streamed astride the second hypnosis induction block and the third hypnosis exit block, the transition from the second sequence of images towards the third sequence of images occurring during the streaming of the second hypnosis induction block.

Said first dominant color might be a warm color, as a red hue, said second dominant color might be a natural color of the virtual reality environment and said third dominant color might be a cold color, as a blue hue.

According to an embodiment, at least a fourth therapeutic components is stored on the storage medium; said fourth therapeutic component comprises an alternative bilateral stimulation video stream representing an object having an alternating motion pattern, and, following the reception of a trigger instruction of the therapeutic method, said controlling unit is configured to simultaneously stream the first, the second, the third and the fourth therapeutic components through the virtual reality head mounted device.

Said alternative bilateral stimulation video stream might be included in said immersive video stream or might be a distinct video stream of said immersive video stream. Preferably, said alternative bilateral stimulation video stream might be superimposed on the virtual reality environment at a predetermined position.

Said object can be a shape (in 2 dimensions) or an object (in 3 dimensions). It can be regular or irregular. Non limiting examples include a dot, a circle, a sphere, a cylinder, a square, a cube, a cuboid, a triangle, a pyramid, a cone, a polygon, etc. It might be an element of the virtual reality environment, or alternatively can be superimposed on top of the images displaying or representing the virtual reality environment.

According to an embodiment, said alternating motion pattern is a rhythmic left-right pattern with a frequency ranging from about 0.2 Hz to about 2 Hz, especially of about 0.8 Hz. In other words, said object moves, swings or oscillates from left to right to left, and so on, and the time necessary for the object to move from centered, to one side, then from the other side back to the center, ranges from about to 0.5 seconds about 5 seconds, especially of about 1.25 seconds.

In some embodiments, graphical trigger elements do not appear and disappear or move around in said virtual reality environment concomitantly with alternating bilateral visual stimulations.

According to an embodiment, the alternative bilateral stimulation video stream comprises at least a first and a second sequence of images each representing an alternative bilateral stimulation, wherein said first sequence of images is to be streamed between the end of the streaming of the first sequence of binaural beats and the beginning of the streaming of the second sequence of binaural beats; and wherein said second sequence of images is to be streamed after end of the streaming of the second sequences of binaural beats.

In some embodiments, the first and/or the second sequence of images lasts of a period of time ranging from about 120 seconds to about 240 seconds, especially of about 180 seconds, or from about 240 seconds to about 480 seconds, especially of about 360 seconds.

In some embodiment, the first and/or the second sequence of images comprises or consists of two or more subsequences interrupted by rest periods, each subsequence comprising or consisting of from 10 to 20 left-right moves of the object, especially of 14 left-right moves of the object. By "rest period", it is meant that the object does not move in a rhythmic left-right pattern and remains still. Alternatively, the object can disappear during the rest period and reappear at the beginning of the following subsequence.

According to an embodiment, the third hypnosis exit bloc is to be streamed with or after the end of the streaming of the second sequence of images.

According to an embodiment, at least a fifth therapeutic components is stored on the storage medium; said fifth therapeutic component comprises a third soundtrack comprising sounds of nature; and, following the reception of a trigger instruction of the therapeutic method, said controlling unit is configured to simultaneously stream the first, the second, the third, the fourth and the fifth therapeutic components through the virtual reality head mounted device.

Examples of sounds of nature include, but are not limited to, water sounds (e.g., ocean waves, flowing river, rainfall, waterfall, gentle stream, etc.), wind sounds, animal sounds (e.g., bird songs, etc.), and combinations thereof.

In some embodiments, the sounds of nature are linked or related to the virtual reality environment described above. By way of example, when the virtual reality environment displays or represents an ocean's shore, the soundtrack comprises sounds of ocean waves, sea wind, shore animals, etc. When the virtual reality environment displays or represents a forest, the soundtrack comprises sounds of wind rustling in trees, forest animals, twig cracking, etc.

In some embodiments, the sound volume of the third soundtrack might be lowered during the streaming of the alternative bilateral stimulation video stream and/or during the streaming of the third hypnosis exit block.

According to an embodiment, during the streaming of the therapeutic components, said controlling unit is configured to modulate the sound volume of the third soundtrack for each left and right speakers of the stereo headphones according to the spatial orientation of the head-mounted display.

Preferably, said head-mounted display might comprise orientation sensors able to sense or monitor the spatial orientation if the head-mounted display.

The subject of the invention is also a virtual reality method intended to be implemented in a therapeutic system according to the invention, said method comprising a step of simultaneously streaming, through the virtual reality head mounted device of the therapeutic system:
a. said immersive video stream stored on the storage medium to generate a virtual reality environment in which the patient is immersed;
b. said first soundtrack stored on the storage medium comprising a first and a second successive sequences of binaural beats;
c. said second soundtrack stored on the storage medium comprising a spoken hypnosis script.

The subject of the invention is also a digital therapeutic method for pain relief, implemented with a therapeutic system according to the invention which is worn by a patient.

Preferably, said digital therapeutic method might comprise a step of simultaneously streaming, through the virtual reality head mounted device of the therapeutic system:
a. said immersive video stream stored on the storage medium to generate a virtual reality environment in which the patient is immersed;
b. said first soundtrack stored on the storage medium comprising a first and a second successive sequences of binaural beats;
c. said second soundtrack stored on the storage medium comprising a spoken hypnosis script.

Preferably, the digital therapeutic method is for pelvic pain relief. In some embodiment, the digital therapeutic method is for chronic pelvic pain relief. In some embodiment, the digital therapeutic method is for endometriosis related pain relief.

The subject of the invention is also a digital non-therapeutic method of relaxation, implemented with a therapeutic system according to the invention which is worn by a user.

The subject of the invention is also a computer program product recorded on a computer storage device, the computer program product including instructions that when executed by a processor cause the processor to execute the steps of the method according to invention or the digital therapeutic method according to invention or the digital non-therapeutic method according to the invention.

The subject of the invention is also a computer-readable storage medium including portions of code of a computer program intended to be executed by the controlling unit of a therapeutic system according to the invention so as to implement the method according to invention or the digital therapeutic method according to invention or the digital non-therapeutic method according to the invention.

According to an embodiment, the computer-readable storage medium further contains at least the first, the second and the third therapeutic components.

The present invention will be better understood, and other advantages will appear on reading the detailed description of an embodiment taken by way of non-limiting example and illustrated by the appended drawings, in which:
**Figure** 1 shows a simplified therapeutic system for the implementation of a therapeutic method for pain relief, according to an embodiment of the invention;
**Figure 2** is a logic flow diagram that depicts the method implemented by the therapeutic system shown in **Figure 1****;**
**Figure** 3 is an instant 2D view of a virtual reality environment provided to the patient during a step of the method shown in **Figure 2****;**
**Figure 4** is an instant 2D view of a virtual reality environment provided to the patient during an other step of the method shown in **Figure 2****;**
**Figure** 5 is a graph showing the mean difference of pain felt by 8 participants between the end of a 60 second pain administration and the end of treatment with either of binaural beats, sounds of nature, an oratorical soundtrack comprising a hypnosis script, alternating bilateral visual stimulations or a virtual reality environment;
**Figure** 6 is a graph showing perceived pain relief rates after the implementation of the method of **Figure 2** and after the implementation of a known digital treatment.

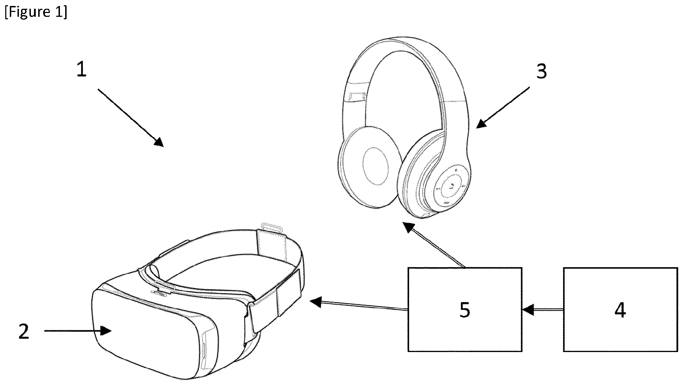

**Figure 1** shows a therapeutic system 1 according to an embodiment of the invention. The system 1 comprises a head-mounted display 2, such as a virtual reality or VR headset, and stereo headphones 3.

### Description of the system

The system 1 also comprises a storage medium 4, which might be a memory of the VR headset 2, a memory of a smartphone in connection, by wire or wireless, with the VR headset 2 or a memory of a distant server with which the VR headset 2 exchanges data.

The system also comprises a controlling unit 5 of the VR headset 2 and the headphones 3, said controlling unit being able to stream immersive video files, stored in the memory 4, through the VR headset 2 and soundtracks, stored in the memory 4, through the headphones 3.

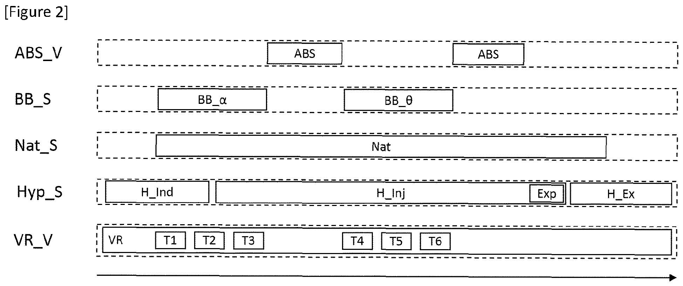

A plurality of digital therapeutic components VR_V, BB_S, Hyp_S, ABS_V, Nat_S are stored in the memory 4, forming together a digital therapeutic treatment for pain relief. These components are depicted on **Figure 2****.**

A first therapeutic component comprises an immersive video stream VR_V intended to generate a virtual reality environment VR.

In the non-limitative present embodiment, when the immersive video stream VR_V is played on the VR headset 2, it generates a virtual reality environment VR representing a lagoon surrounded by a forest.

The immersive video stream comprises a six successive subsets of images T1 to T6, which each represents the appearance and the disappearance of a graphical trigger element in said virtual reality environment VR. The trigger element of subset T1 is a shooting star sailing through the sky, the trigger element of subset T2 is a cloud of fireflies flying away, the trigger element of subset T3 is a bubble that bursts into a sphere, the trigger element of subset T4 is a rainbow, the trigger element of subset T5 is a flower that opens and the trigger element of subset T6 is a bubble that bursts into a sphere. The number and/or the nature of the graphical trigger elements might be different.

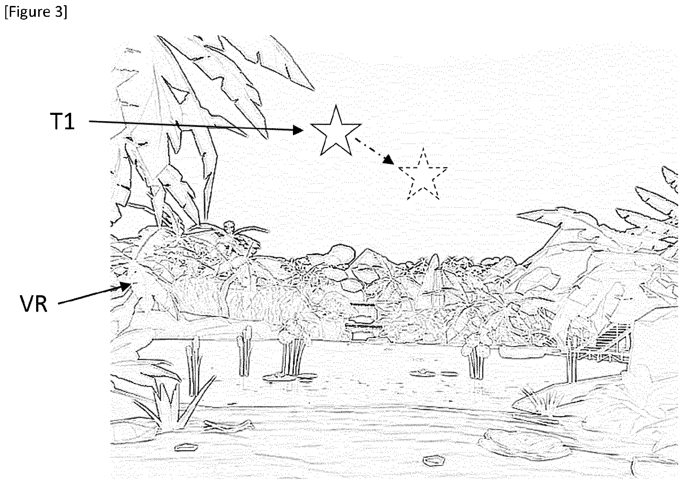

The virtual reality environment VR with the trigger element of subset T1 has been depicted on **Figure 3****.**

A second therapeutic component comprises a first soundtrack BB_S comprising a first and a second successive sequences of binaural beats.

The first sequence of binaural beats consists of an alpha binaural beats sequence BB_α comprising two dichotic pure-tone sine waves, with a frequency of 466 Hz for the left pure-tone sine wave and 456 Hz for the right pure-tone sine wave, the frequency difference being of 10 Hz.

The second sequence of binaural beats comprises or consists of a theta binaural beats sequence BB_θ, following said alpha binaural beats sequence BB_α and comprising two dichotic pure-tone sine waves, with a frequency of 466 Hz for the left pure-tone sine wave and 460 Hz for the right pure-tone sine wave, the frequency difference being of 6 Hz.

Each of the alpha binaural beats sequence BB_α and the beta binaural beats sequence BB_θ lasts of a period of time of 240 seconds.

In the depicted example, the first soundtrack BB_S comprises a period of time of 180 seconds, separating the alpha binaural beats sequence BB_α and the beta binaural beats sequence BB_θ, which does not contain any sound. As a variant, each sequence of binaural beats BB_α and BB_θ might be stored in the memory 4 as a sub soundtrack, each sub soundtrack being thus associated with a time index indicating its start time in the soundtrack BB_S.

A third therapeutic component comprises a second soundtrack Hyp_S comprising a hypnosis script, spoken by a woman with a voice frequency ranging from about 150 to about 300 Hz.

Said hypnosis script comprises a first hypnosis induction block H_Ind of predefined sentences ; a second hypnosis injunction block H_Inj of predefined sentences; and a third hypnosis exit block H_Ex of predefined sentences.

In the depicted example, the first block H_Ind includes a spoken welcoming subsequence and a spoken induction subsequence, the second block H_Inj includes a spoken dissociation subsequence, a spoken questioning subsequence, a spoken sensory openness subsequence, and a spoken post hypnotic suggestion subsequence. More specifically, the second block H_Inj comprises three requests Exp to the user asking him to breath in and out.

Each sentence of the second hypnosis injunction block H_Inj, and optionally of the first hypnosis induction block H_Ind, is time-spaced from the previous sentence of a period of time ranging from about 2 seconds to 5 seconds.

The first hypnosis induction block H_Ind lasts of a period of time of about 130 seconds, the second hypnosis injunction block H_Inj lasts of a period of about 1080 seconds and the third hypnosis exit block H_Ex lasts of a period of time of about 120 seconds.

To enhance the hypnosis injunction, the voice rhythm and/or the voice pitch and/or the voice loudness and/or the voice harmonic to noise ratio and/or the voice frequency decreases for each subsequence of the second hypnosis injunction block H_Inj.

A fourth therapeutic component comprises an alternative bilateral stimulation video stream ABS_V representing an object having an alternating motion pattern. As a variant, said fourth therapeutic component might be included into the immersive video stream VR_V.

The alternative bilateral stimulation video stream ABS_V comprises a first and a second sequence of images ABS each representing an alternative bilateral stimulation

For each sequence ABS, said object is the sphere formed by the burst of the bubble of subsets of images T3 and T6 of the immersive video stream VR_V. Thus, no graphical trigger element appears, nor disappears or moves around in said virtual reality environment VR concomitantly with alternating bilateral visual stimulations ABS. As a non-described embodiment of the invention, the object might be formed by the final shape of an other trigger element or by an object unrelated to a trigger element.

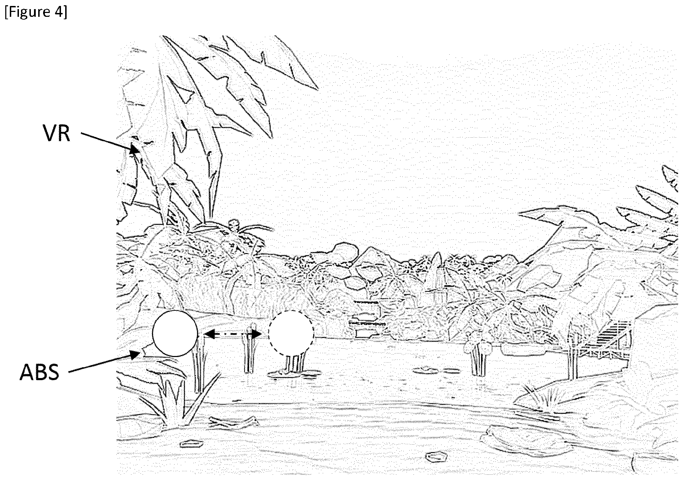

A sequence ABS superimposed on the virtual reality environment VR has been depicted on **Figure 4****.**

Each sequence ABS comprises three subsequences of alternating motion pattern, interrupted by rest periods, each subsequence comprising or consisting of from 10 to 20 left-right moves of the object, especially of 14 left-right moves of the object. For each subsequence, said alternating motion pattern is a rhythmic left-right pattern with a frequency of about 0.8 Hz.

Each sequence of images ABS lasts of a period of time ranging of about 180 seconds. In the depicted example, the alternative bilateral stimulation video stream ABS_V comprises a period of time of 240 seconds, separating the sequences ABS, which does not contain any stimulation. As a variant, each sequence ABS might be stored in the memory 4 as a sub video stream, each sub video stream being thus associated with a time index indicating its start time in the video stream ABS_V.

A fifth therapeutic component comprises a third soundtrack Nat_S comprising sounds of nature.

### Description of the treatment

According to the invention, the system 1 implements a digital therapeutic treatment for women suffering from pelvic pain related with endometriosis. An application is stored on the VR headset 2, or on the control unit 5, to allow a patient to start the treatment.

On a preliminary step, the patient wears the VR headset 2 and the headphones 3, and trigger the treatment, for instance with pushing, clicking, or pointing on a button in the application.

Following the reception of the trigger instruction, the control unit simultaneously streams the digital therapeutic components VR_V, BB_S, Hyp_S, ABS_V, Nat_S stored in the memory 4. **Figure 2** shows an exemplary timeline of these digital therapeutic components along the administration of the treatment.

The immersive video VR_V is streamed through the headset 2, causing the patient to be immersed in the virtual reality environment VR, while the first hypnosis induction block H_Ind is streamed through the headphones 3, welcoming the patient and inducing a hypnosis state to the patient.

The first hypnosis induction block H_Ind is followed with the second hypnosis injunction block H_Inj, to cause a trance or a mind dissociation to the patient.

Concomitantly, the first sequence of binaural beats BB_α is streamed through the headphones 3, astride the first hypnosis induction block H_Ind and the second hypnosis injunction block H_Inj. Simultaneously, the third soundtrack Nat_S is also streamed through the headphones 3.

The subsets of images T1, T2 and T3 are streamed in the virtual reality environment VR, through the headset 2, concomitantly with the first sequence of binaural beats BB_α.

The first hypnosis induction block H_Ind and/or the second hypnosis injunction block H_Inj includes sentences inviting the user to look at an area of the virtual reality environment VR, for instance a left, a top, a right, or a bottom area. The graphical trigger elements of subsets of images T1, T2 and T3 appear in said area. Graphical trigger elements avoid any monotony and boredom of the patient immersed in the virtual reality environment VR.

At the end of the alpha binaural beats sequence BB_α, the first sequence ABS is streamed in the virtual reality environment VR, through the headset 2, while no more binaural beats can be heard by the patient. Moreover, during each subsequences of alternating motion pattern of the sequence ABS, no sentences is spoken and the sound volume of the third soundtrack Nat_S is lowered.

At the end of the first sequence ABS, the second sequence of binaural beats BB_θ is streamed through the headphones 3, during the second hypnosis injunction block H_Inj.

The subsets of images T4, T5 and T6 are streamed in the virtual reality environment VR, through the headset 2, concomitantly with the first sequence of binaural beats BB_θ.

At the end of the theta binaural beats sequence BB_ θ, the second sequence ABS is streamed in the virtual reality environment VR, through the headset 2, while no more binaural beats can be heard by the patient.

As depicted on **Figure 2****,** ABS sequences, alpha binaural beats sequence BB_α and beta binaural beats sequence BB_θ have no overlap with each other, so as to help the patient to fully focus on once stimulation at every moment.

At the end of the second sequence ABS, the requests Exp are streamed. The breath in and out of the patient helps her to enhance the benefits of the hypnosis and to prepare the exit of the trance.

The third hypnosis exit bloc H_Ex is streamed after the end of the request Exp.

The sound volume of the third soundtrack Nat_S is lowered during the streaming of the third hypnosis exit block H_Ex and is eventually shut off.

In the embodiment that has been described, the digital therapeutic components VR_V, BB_S, Hyp_S, ABS_V, Nat_S are designed to trigger stimulations or events at the right instant. According to an alternative embodiment of the invention, the control unit might trigger each stimulation or event at a predetermined instant, to achieve the treatment of **Figure 2.**

### Experiment 1

The aim of this experiment was to define the therapeutic potential of each element of the digital therapeutic composition taken individually.

Eight healthy participants (5 women, 3 men, from 24 to 36 years old) were exposed to a conditioned pain modulation paradigm by putting their hands in cold water for 60 seconds. The participants were then subjected to binaural beats, sounds of nature, an oratorical soundtrack comprising a hypnosis script, alternating bilateral visual stimulations or a virtual reality environment.

Pain was rated at the end of the 60 second immersion in water, and after treatment (subjection to each of the digital therapeutic composition elements taken individually).

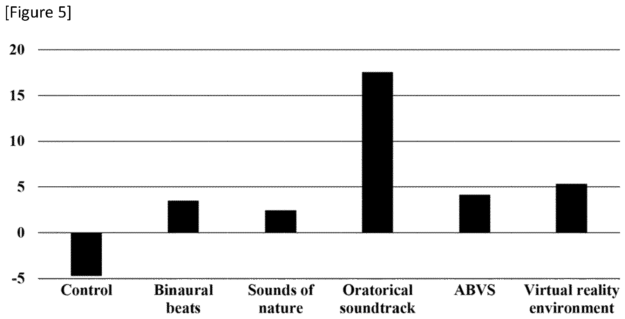

Results are shown in **Figure 5****,** representing the mean difference of pain felt by the participants between the end of the 60 second immersion in water and the end of treatment.

As seen in this figure, the oratorical soundtrack comprising a hypnosis script provided by far the best therapeutic efficacy for alleviating pain. Binaural beats, sounds of nature, alternating bilateral visual stimulations and virtual reality environment also showed a therapeutic efficacy for alleviating pain, although to a lesser extent.

Conversely, when the participants were not subjected to any treatment after their 60 second immersion in water (control), pain further amplified.

### Experiment 2

This was a randomized, controlled, comparative, open label, two parallel group, interventional study comparing the effect of the digital therapeutic treatment described herein (and hereafter named "Endocare") and a digital control on endometriosis related pain after a single use. This randomized controlled trial was conducted between December 2020 and May 2021 in Clinic Tivoli Ducos in Bordeaux, France.

The Endocare treatment developed by the Inventors was displayed through a VR headset (Oculus Quest) associated with a high-quality headphone (APK K 240 MKII). Endocare offers a 20-minute treatment consisting of a combination of auditory and visual therapeutic procedures integrated in a 3D VR environment.

The digital control was developed specifically for this study. It was displayed through a tablet (Samsung Galaxy Tab A) associated with a high-quality headphone similar to those used in the Endocare group (APK K 240 MKII). The digital comparator was a 20-minute control with the same composition as the Endocare treatment (same context, same environment, same duration) but without visual and auditory immersion. A soundtrack composed of nature sounds related to the projected image.

45 subjects were included and randomized in this trial to one of the two groups, Endocare or control. The treatment (either Endocare or control) consisted in a single use.

All subjects were diagnosed with endometriosis by specialized gynecologists. Their pain level needed to be at least 4 on a 0 to 10 numeric rating scale (NRS) to be able to participate to the study. This pain intensity evaluation represents the pretreatment evaluation just before the use of Endocare or control and was considered as baseline (T0).

The follow up duration lasted for 240 minutes (T240) following the treatment with Endocare or control.

Once the treatment was completed, the patient was asked to rate on a paper questionnaire the pain relief on a 5 points categorical scale (0 = no relief, 1 = slight relief, 2 = moderate relief, 3 = important relief, 4 = complete relief) and pain intensity based on the 11 points NRS (0 = no pain; 10 = unbearable pain) at 15 minutes (T15), 30 minutes (T30), 45 minutes (T45), 60 minutes (T60) and 240 minutes (T240) after administration of the allocated study treatment.

A comparison was done one the difference between the baseline pain (T0) and the clustered five post treatment measurements covering 15 minutes to 4 hours after the treatment (T15, T30, T45, T60, T240). Both the treatment (Endocare) and the control groups significantly reduced the pain perception (Endocare: mean reduction = 1.38, p = 0.001; Control: mean reduction = 0.58, p = 0.008). The group (Endocare, Control) x times (T0, post all) was also significantly different (F = 7.343, p = 0.010), Endocare being significantly more efficient than the control.

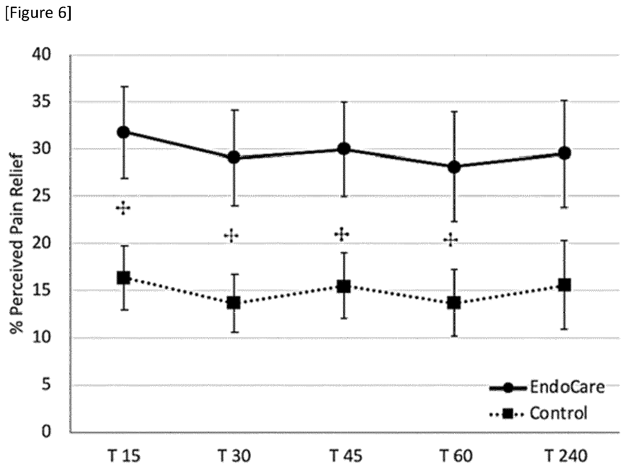

At each post treatment measurement, the patients were asked to measure their perceived pain relief on a 0 to 4 numerical scale from no relief to total relief. The mean pain relief reported for Endocare was 28 % (range: 26 to 30 %) versus 15 % for the control (range: 14 to 16 %). Nonparametric Wilcoxon unilateral unpaired tests corrected for multiple comparisons with FDR were performed. All post treatment times presented a significantly higher pain relief score for Endocare compared to the control group (p < 0.05) (Figure 6).

The mean maximum effect was 42 % (95 % CI: 30.82; 53.18) for Endocare and 22 % (95 % CI: 15.38; 28.53) for the control group. The maximum effect was significantly higher for the treatment group (p = 0.004).

In this randomized controlled trial, placebo control study, we aimed at measuring the immediate and persisting effect of a DTx (Endocare) on pain in women suffering from pelvic pain related with endometriosis.

We hypothesized that Endocare would be superior to the control at reducing clinical pain perception. Both Endocare and the control reduced the overall pain perception when comparing the pain at baseline (T0) with the combined five post treatment measurements from 15 minutes (T15) to 4 hours (T240).

However, Endocare was significantly superior to the control. The mean effect was 1.38 for Endocare and 0.58 for the control. The percent of the perceived pain reduction ranged between 26 % to 30 % for Endocare, and from 14 % to 16 % for the control. These results were partially explained by the cohort of our study, that comprised women with severe endometriosis.

Previous studies had shown that a virtual reality environment was an effective way to reduce acute pain intensity, especially pain experienced during medical procedures, burn victims, or by women during childbirth. Some studies have also looked at the effects of VR on chronic pain, and reported that VR was effective to reduce, e.g., chronic low back pain throughout the duration of the treatment.

In our study, to potentialize VR effects, we chose additional auditory and visual stimulations, to offer a DTx treatment that could act on different components of pain: alpha and theta binaural beats, sounds of nature, alternating bilateral visual stimulations, and hypnosis.

The combination of these stimulations coupled with the VR experience led to the decrease in pain observed in the Endocare group immediately after and up to 4 hours post treatment.

To our knowledge, our study is the first to demonstrate an effect on pain reduction in women diagnosed and suffering from moderate to severe endometriosis related pelvic pain using a non-pharmacological treatment combining VR with visual and auditory stimuli.

The illustrations described herein are intended to provide a general understanding of the structure of various embodiments. These illustrations are not intended to serve as a complete description of all of the elements and features of apparatus, processors and systems that utilizes the structures or methods described therein. Many other embodiments or combinations thereof may be apparent to those of ordinary skills in the art upon reviewing the disclosure by combining the disclosed embodiments. Other embodiments may be utilized and derived from the disclosure, such that structural and logical substitutions and changes may be made without departing from the scope of the disclosure.

Further, the disclosure and the illustrations are to be considered as illustrative rather than restrictive, and the appended claims are intended to cover all such modifications, enhancements and other embodiments, which fall within the true spirit and scope of the description. Thus, the scope of the following claims is to be determined by the broadest permissible interpretation of the claims and their equivalents and shall not be restricted or limited by the foregoing description.

## Claims

1. Therapeutic system for the implementation of a therapeutic method for pain relief, comprising a virtual reality head mounted device having at least a head-mounted display and stereo headphones; said system comprising at least a storage medium and a controlling unit of the virtual reality head mounted device, wherein at least a first, a second and a third therapeutic components are stored on the storage medium; wherein:
a. said first therapeutic component comprises an immersive video stream intended to generate a virtual reality environment in which a patient has to be immersed;
b. said second therapeutic component comprises a first soundtrack comprising a first and a second successive sequences of binaural beats, the beats frequencies differences being different from one binaural beats sequence to the other;
c. said third therapeutic component comprises a second soundtrack comprising a spoken hypnosis script;
and wherein, following the reception of a trigger instruction of the therapeutic method, said controlling unit is configured to simultaneously stream the first, the second and the third therapeutic components through the virtual reality head mounted device.

2. Therapeutic system according to claim **1,** wherein the first sequence of binaural beats comprises an alpha binaural beats sequence comprising two dichotic pure-tone sine waves, with a frequency difference ranging from about 9 to about 14 Hz; and wherein the second sequence of binaural beats comprises a theta binaural beats sequence following said alpha binaural beats sequence and comprising two dichotic pure-tone sine waves, with a frequency difference ranging from about 4 to about 8 Hz.

3. Therapeutic system according to claim **1** or **2**, wherein the second soundtrack comprises:
a. a first hypnosis induction block of predefined sentences spoken by a human voice;
b. a second hypnosis injunction block of predefined sentences spoken by a human voice; and
c. a third hypnosis exit block of predefined sentences spoken by a human voice.

4. Therapeutic system according to claim **3,** wherein each block is spoken by a same woman **characterized by** a voice frequency ranging from about 150 to about 300 Hz.

5. Therapeutic system according any one of claims **3** to **4,** wherein the voice rhythm and/or the voice pitch and/or the voice loudness and/or the voice harmonic to noise ratio decreases along the second hypnosis injunction block.

6. Therapeutic system according to any one of claims **3** to **5,** wherein the first sequence of binaural beats is to be streamed astride the streaming of the first hypnosis induction block and the second hypnosis injunction block; and the second sequence of binaural beats is to be streamed during the streaming of the second hypnosis injunction block.

7. Therapeutic system according to any one of claims **1** to **6,** wherein the immersive video stream comprises a sequence of images representing said virtual reality environment and in which at least a subset of images represents the appearance and the disappearance of a graphical trigger element in said virtual reality environment.

8. Therapeutic system according to claim **7,** wherein the first or the second sequence of binaural beats is to be streamed simultaneously with the streaming of said subset of images.

9. Therapeutic system according to claims **7** or **8** in combination with any one of claims **3** to **6,** wherein the immersive video stream comprises a first sequence of images representing said virtual reality environment with a first dominant color and a second sequence of images, immediately following the first sequence of images and representing said virtual reality environment with a second dominant color distinct from the first dominant color, and wherein the first sequence of binaural beats is to streamed with or after the beginning of the streaming the second sequence of image.

10. Therapeutic system according to any one of claims **1** to **9,** wherein at least a fourth therapeutic components is stored on the storage medium; wherein said fourth therapeutic component comprises an alternative bilateral stimulation video stream representing an object having an alternating motion pattern, and wherein, following the reception of a trigger instruction of the therapeutic method, said controlling unit is configured to simultaneously stream the first, the second, the third and the fourth therapeutic components through the virtual reality head mounted device.

11. Therapeutic system according to claim **10,** wherein said alternating motion pattern is a rhythmic left-right pattern with a frequency ranging from about 0.2 Hz to about 2 Hz.

12. Therapeutic system according to claims **10** or **11,** wherein the alternative bilateral stimulation video stream comprises at least a first and a second sequence of images each representing an alternative bilateral stimulation, wherein said first sequence of images is to be streamed between the end of the streaming of the first sequence of binaural beats and the beginning of the streaming of the second sequence of binaural beats; and wherein said second sequence of images is to be streamed after end of the streaming of the second sequences of binaural beats.

13. Therapeutic system according to claim **12** in combination with any one of claims **3** to **6,** wherein the third hypnosis exit bloc is to be streamed with or after the end of the streaming of the second sequence of images.

14. Therapeutic system according to any one of claims **1** to **13,** wherein at least a fifth therapeutic components is stored on the storage medium; wherein said fifth therapeutic component comprises a third soundtrack comprising sounds of nature; and wherein, following the reception of a trigger instruction of the therapeutic method, said controlling unit is configured to simultaneously stream the first, the second, the third, the fourth and the fifth therapeutic components through the virtual reality head mounted device.

15. Therapeutic system according to claim **14,** wherein, during the streaming of the therapeutic components, said controlling unit is configured to modulate the sound volume of the third soundtrack for each left and right speakers of the stereo headphones according to the spatial orientation of the head-mounted display.

16. Virtual reality method intended to be implemented in a therapeutic system according to any one of claims **1** to **15,** said method comprising a step of simultaneously streaming, through the virtual reality head mounted device of the therapeutic system:
a. said immersive video stream stored on the storage medium to generate a virtual reality environment in which the patient is immersed;
b. said first soundtrack stored on the storage medium comprising a first and a second successive sequences of binaural beats;
c. said second soundtrack stored on the storage medium comprising a spoken hypnosis script.

17. Computer program product recorded on a computer storage device, the computer program product including instructions that when executed by a processor cause the processor to execute the steps of a method according to the claim **16.**

18. Computer-readable storage medium including portions of code of a computer program intended to be executed by the controlling unit of a therapeutic system according to any one of claims **1** to **15** so as to implement the method according to claim **16.**

19. Computer-readable storage medium according to claim **18,** further containing at least the first, the second and the third therapeutic components.
